(19) Europäisches Patentamt
European Patent Office
Office européen des brevets

(11) Veröffentlichungsnummer: **0 383 162 A1**

(12) # EUROPÄISCHE PATENTANMELDUNG

(21) Anmeldenummer: **90102342.4**

(22) Anmeldetag: **07.02.90**

(51) Int. Cl.$^5$: **A61K 31/355, A61K 31/60, //(A61K31/355,31:19), (A61K31/60,31:355,31:19)**

(30) Priorität: **16.02.89 DE 3904674**

(43) Veröffentlichungstag der Anmeldung: **22.08.90 Patentblatt 90/34**

(84) Benannte Vertragsstaaten: **AT BE CH DE DK FR GB IT LI**

(71) Anmelder: **CASSELLA Aktiengesellschaft**
**Hanauer Landstrasse 526**
**D-6000 Frankfurt am Main 61(DE)**

(72) Erfinder: **Greve, Rainer, Dr.**
**Konrad-Adenauer-Ring 52**
**D-2360 Bad Segeberg(DE)**
Erfinder: **Greve, Harald, Dr.**
**Im Weidenthal 36**
**D-5064 Rösrath 3 (Forsbach)(DE)**
Erfinder: **Pfadt, Joachim**
**Meiereistrasse 36**
**D-2361 Todesfelde(DE)**

(74) Vertreter: **Urbach, Hans-Georg, Dr. et al**
**Hanauer Landstrasse 526**
**D-6000 Frankfurt am Main 61(DE)**

(54) **Verwendung einer Wirkstoffkombination zur Behandlung rheumatischer Erkrankungen.**

(57) Die Wirkstoffkombination aus Vitamin E und Ibuprofen wird zur äußerlichen Behandlung von rheumatischen Erkrankungen verwendet.

EP 0 383 162 A1

## Verwendung einer Wirkstoffkombination zur Behandlung rheumatischer Erkrankungen

Die Erfindung betrifft die Verwendung einer Wirkstoffkombination aus Vitamin E und/oder einem oder mehreren Vitamin-E-Derivaten und Ibuprofen (2-(4-Isobutylphenyl)-propionsäure) und/oder einem oder mehreren Salzen des Ibuprofens, gegebenenfalls mit Zusatz von Salicylsäure und/oder einem oder mehreren Salicylsäurederivaten zur topischen, d.h. äußerlichen Behandlung von rheumatischen Erkrankungen, d.h. akut-entzündlicher und chronisch-entzündlicher Prozesse des rheumatischen Formenkreises in Gelenken und Weichteilen des Menschen.

Nach neuerer medizinisch-wissenschaftlicher Erkenntnis werden der unkontrollierte Abbau von Arachidonsäure und die in der Folge gebildeten Abbauprodukte in den betroffenen Geweben als Hauptursache des rheumatisch-entzündlichen Prozesses angesehen. Der Abbau vollzieht sich auf zwei getrennten Reaktionswegen (in der Folge Weg 1 und Weg 2 genannt), da die Arachidonsäure das Substrat für die zwei Enzyme Cyclooxygenase (Weg 1) und Lipoxygenase (Weg 2) ist. Abbauprodukte sind die Prostaglandine (Weg 1) und die Leukotriene (Weg 2); beide Substanzgruppen gelten als Mediatoren für rheumatisch-entzündliche Prozesse im Organismus.

Dis bisher verfügbaren antirheumatisch wirksamen Substanzen lassen sich in der Hauptsache in zwei größere Gruppen aufteilen, nämlich in die hormonartigen bzw. steroidalen und in die nichtsteroidalen Antirheumatica. Zu den hormonartigen bzw. steroidalen Antirheumatica zählen z.B. Corticosteroide, wie z.B. Cortison, Hydrocortison, Prednison und Prednisolon. Zu den nichtsteroidalen Antirheu matica zählen Salicylsäure und Salicylsäurederivate sowie Ibuprofen, 2-(4-Isobutylphenyl)-propionsäure. Daneben werden in einzelnen Fällen z.B. noch Goldpräparate, Penicillamin, Zytostatica, hydrolytische Enzyme und Immunmodulatoren zur Behandlung rheumatischer Erkrankungen eingesetzt.

Während die steroidalen Antirheumatica wegen zahlreicher Nebenwirkungen der Rezeptpflicht unterliegen und nur unter ärztlicher Kontrolle angewendet werden, stehen zur freien Verfügung des Patienten lediglich einige Substanzen mit nichtsteroidaler chemischer Strukur bereit. In den meisten Fällen handelt es sich bei diesen um Ibuprofen oder Salicylsäure bzw. ihre Derivate, die allein oder kombiniert mit anderen chemischen Wirkstoffen, pflanzlichen Extrakten oder ätherischen Ölen, innerlich oder äußerlich zur Behandlung rheumatischer Erkrankungen angewendet werden.

Nach derzeit vorliegenden Erkenntnissen hemmen das Ibuprofen und die Salizylsäure und ihre Derivate lediglich die Prostaglandinbildung aus Arachidonsäure (Weg 1). Um eine sichere Wirkung, vor allem bei chronischen Formen von Rheuma, zu erzielen, ergibt sich, inbesondere bei ihrer innerlichen Verabreichung, eine zwangsläufig hohe Dosierung mit bei Daueranwendern steigender Tendenz. Negative Auswirkungen auf den Verdauungstrakt, z.B. Magenblutungen, unter Umständen mit Geschwürbildung, sind bei fast allen nichtsteroidalen Antirheumatica medizinisch belegt und als Risikofaktoren anzusehen.

Zur Hemmung der Leukotrienbildung (Weg 2) werden im Markt einige höherdosierte Vitamin-E-Präparate, alle zum Einnehmen, angeboten. Eine topische Anwendung von Vitamin E zur Behandlung von rheumatischen Entzündungen wurde bis her nicht verwirklicht.

Die antirheumatische und schmerzlindernde Wirkung des Ibuprofens oder der Salicylsäure und ihrer Derivate ist, zumal bei topischer Anwendung, nur mäßig ausgeprägt. Entsprechendes gilt nach durchgeführten Versuchen auch für die topische Anwendung von Vitamin E und seinen Derivaten.

Es wurde nun überraschenderweise gefunden, daß eine Wirkstoffkombination von Vitamin E und/oder einem oder mehreren Vitamin-E-Derivaten mit Ibuprofen und gegebenenfalls einem Zusatz von Salicylsäure und/oder einem oder mehreren Salicylsäurederivaten bei topischer Anwendung eine unerwartet starke, d.h. synergistische, den Cortisonen vergleichbare, antirheumatische, entzündungshemmende und schmerzstillende Wirkung entfaltet.

Demgemäß betrifft die Erfindung die Verwendung einer Wirkstoffkombination von Vitamin E und/oder einem oder mehreren Vitamin-E-Derivaten mit Ibuprofen, sowie gegebenenfalls einem Zusatz von Salicylsäure und/oder einem oder mehreren Salicylsäurederivaten zur topischen Behandlung von rheumatischen Erkrankungen.

In der Wirkstoffkombination kann das Ibuprofen in seiner freien Form, als 2-(4-Isobutylphenyl)-propionsäure und/oder in Form seiner Salze, z.B. als Lysinsalz oder Aluminiumsalz, verwendet werden, wobei aus pharmakologischen Gründen der freien Säure-Form Vorrang zu geben wäre. Die Wirkstoffkombination kann auch Mischungen verschiedener Salze des Ibuprofens oder Mischungen eines oder mehrerer Ibuprofensalze mit der freien Säureform des Ibuprofens enthalten.

Die Salicylsäure kann in freier Form oder insbesondere in Form eines pharmakologisch annehmbaren Salzes mit einer anorganischen und/oder organischen Base vorliegen. Geeignete Salze sind z.B. das Lithium-, Magnesium-, Calcium-, Aluminium-, insbesondere das Natriumsalz, ferner z.B. Cholinsalicylat und

Cholin-magnesium-tri(salicylat). Als weitere Salicylsäurederivate, die in der Wirkstoffkombination vorliegen können, sind z.B. zu nennen: Acetylsalicylsäure (=2-Acetoxybenzoesäure) und ihre pharmakologisch annehmbaren Salze, wie z.B. das Lithium-, Magnesium-, Calcium-, oder Aluminiumsalz: Lysinmonoacetylsalycylat; Salicylsäureester und Salicylamid.

Geeignete Salicylsäureester sind z.B. solche, die durch Veresterung der Salicylsäure mit Alkanolen mit 1 bis 5 C-Atomen entstehen, wie z.B. Salicylsäure-methylester. Geeignet ist auch der Salicylsäurephenylester und vor allem der Salicylsäure-2-hydroxyethylester (=Ethylenglykolmonosalicylat). Der Salicylsäure-2-hydroxyethylester ist ganz besonders bevorzugt.

Neben Salicylamid sind auch Salicylamide geeignet, deren Hydroxylgruppe durch eine Alkoxygruppe mit 1 bis 5 C-Atomen ersetzt ist, z.B. 2-Ethoxybenzamid.

In der Wirkstoffkombination kann neben Ibuprofen und/oder seinen Salzen die Salicylsäure allein oder in Mischung mit einem oder mehreren Salicylsäurederivaten vorliegen. Ebenso kann neben Ibuprofen und/oder seinen Salzen auch ein Salicylsäurederivat allein oder in Mischung mit anderen Salicylsäurederivaten vorliegen.

Vitamin E kommt in der Natur in Samenölen und Keimen von Samen als Gemisch mehrerer strukturähnlicher Verbindungen vor. Im Rahmen der vorliegenden Erfindung werden unter dem Begriff Vitamin-E-Substanzen mit Vitamin-E-Wirkung verstanden, die sich vom Tocol der nachstehenden Formel I (mit $R^1$ = $R^2$ = $R^3$ = H) oder vom Tocotrienol der nachstehenden Formel II (mit $R^1$ = $R^2$ = $R^3$ = H) ableiten. Die wichtigsten dieser Substanzen sind unter den Formeln I und II angegeben.

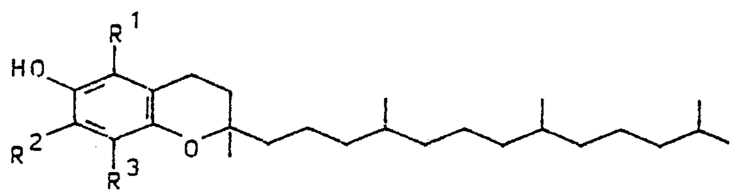

(I)

(Ia), α-Tocopherol: $R^1 = R^2 = R^3 = CH_3$,

(Ib), β-Tocopherol: $R^1 = R^3 = CH_3$, $R^2 = H$,

(Ic), γ-Tocopherol: $R^1 = H$; $R^2 = R^3 = CH_3$,

(Id), δ-Tocopherol: $R^1 = R^2 = H$, $R^3 = CH_3$,

(Ie), ε-Tocopherol: $R^1 = CH_3$, $R^2 = R^3 = H$

(If), Zeta-Tocopherol: $R^1 = R^2 = CH_3$, $R^3 = H$,

(Ig), Eta-Tocopherol: $R^1 = R^3 = H$, $R^2 = CH_3$.

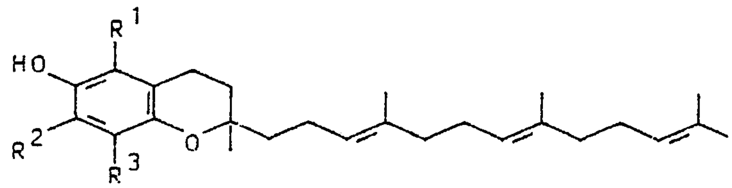

(II)

(IIa), α-Tocotrienol: $R^1 = R^2 = R^3 = CH_3$,

(IIb), β-Tocotrienol: $R^1 = R^3 = CH_3$, $R^2 = H$,

(IIc), γ-Tocotrienol: $R^1 = H$; $R^2 = R^3 = CH_3$,

(IId), δ-Tocotrienol: $R^1 = R^2 = H$, $R^3 = CH_3$,

Die Tocopherole besitzen jeweils 3 Chiralitätszentren und kommen dabei in verschiedenen Konfigurationen vor. Auch bei den Tocotrienolen sind verschiedene Konfigurationen möglich.

Häufig wird das α-Tocopherol als das Vitamin E bezeichnet.

Die Tocopherole und Tocotrienole besitzen unterschiedliche biologische Vitamin-E-Wirkung. Die stärksten biologischen Vitamin-E-Wirkungen besitzen die verschieden konfigurierten α-Tocopherole und ihre Derivate, wie Acetate und Succinate. Die α-Tocopherole und ihre Derivate werden dabei im Rahmen der vorliegenden Erfindung besonders bevorzugt.

Die vorstehend genannten Verbindungen mit Vitamin-E-Wirkung können auch in Form pharmakologisch annehmbarer Derivate in der Wirkstoffkombination vorhanden sein. Geeignete Derivate sind z.B. Ester mit pharmakologisch annehmbaren Mono- oder Dicarbonsäuren, wie z.B. das bevorzugte α-Tocopherolacetat und der bevorzugte Halbester von α-Tocopherol und Bernsteinsäure.

In der Wirkstoffkombination können auch zwei und mehr Verbindungen mit Vitamin-E-Wirkung in freier Form und/oder in Form von Derivaten vorhanden sein.

Die Wirkstoffkombination enthält vorzugsweise α-Tocopherol und/oder ein α-Tocopherol-Derivat und Ibuprofen und/oder ein oder mehrere Salze des Ibuprofens, gegebenenfalls mit einem Zusatz an Ethylenglycolsalicylat.

In der Wirkstoffkombination kann das Gewichtsverhältnis zwischen (Vitamin E und/oder einem oder mehreren Vitamin E-Derivaten) und (Ibuprofen und/oder einem oder mehreren seiner Salze, gegebenenfalls mit Zusatz von Salicylsäure und/oder einem oder mehreren Salicylsäurederivaten) beispielsweise 1 : (0,025 bis 40), vorzugsweise 1 : (0,25 bis 4) und ganz besonders bevorzugt 1 : (0,5 bis 2), betragen. Dabei kann das Gewichtsverhältnis zwischen Ibuprofen und/oder einem oder mehreren seiner Salze einerseits und der Salicylsäure und/oder einem oder mehreren Salicylsäurederivaten andererseits in weiten Grenzen schwanken. Beispielsweise kann das Gewichtsverhältnis (Ibuprofen und/oder einem oder mehreren seiner Salze) : (Salicylsäure und/oder einem oder mehreren Salicylsäurederivaten) = 1 : (0 bis 200), vorzugsweise 1 : (0 bis 10) und ganz besonders bevorzugt 1 : (0 bis 5) betragen.

Die Wirkstoffkombination wird durch Mischen der Einzelwirkstoffe in den genannten Gewichtsverhältnissen hergestellt. Um Überdosierungen und damit möglicherweise verbundene Sekundär- und Nebenwirkungen, z.B. Hautreizungen bei der Anwendung, zu vermeiden, ist es zweckmäßig, die Wirkstoffkombination bzw. die Einzelwirkstoffe in inerte Trägerstoffe einzuarbeiten und sie in Form pharmazeutischer Zubereitungen anzuwenden. Durch die Wahl geeigneter Trägerstoffe kann gleichzeitig auch das Eindringvermögen der Wirksubstanzen in die Haut bedeutend gesteigert werden.

Die pharmazeutischen Zubereitungen für die topische Anwendung können z.B. fest, halbfest oder flüssig sein. Sie können z.B. Puder, Fett-Pasten, Salben, Cremes oder Schüttelmixturen sein, oder auch in Form von Salbenstiften oder Pflastern angewandt werden.

Die Herstellung der pharmazeutischen Zubereitungen erfolgt in an sich bekannter Weise durch Mischen der Trägerstoffe mit einer pharmakologisch wirksamen Menge der Wirkstoffkombination.

Zur Herstellung von Pudern können z.B. als Trägerstoffe verwendet werden: Zinkoxyde, Talcum, Calciumkarbonat, Zinkcarbonat, Diatomeenerde, Aluminiumoxid, Aluminiumsilicat, Zinkstearat, Maisstärke, Zellulosederivate wie Methylcellulose und Carboxymethylcellulose, Gelatine. Zur Herstellung von Pudern eignen sich besonders feste Wirkstoffe, wie z.B. der Halbester von α-Tocopherol und Bernsteinsäure.

Als Trägerstoffe für die Herstellung von flüssigen Darreichungsformen, wie Lösungen, Emulsionen oder Linimenten, eignen sich z.B. alle natürlichen und synthetischen neutralen fetten Öle, pharmazeutischen Emulgatoren, Wasser, wäßrigen Gele wie Polyacrylatgel, alle hautverträglichen Alkohole, Glycerin und andere Polyole.

Als Trägerstoffe für die Herstellung von halbfesten, pharmazeutischen Zubereitungen, wie z.B. Salben, Cremes, Gelen oder Pasten eignen sich z.B. Paraffinkohlenwasserstoffe, Vaseline, pflanzliche Öle und tierische Fette, synthetische Glyzeride, Wollwachsprodukte, Wachse, flüssige Polyalkylsiloxane und pharmazeutisch verwendbare, viskositätserhöhende Grundstoffe.

Trägerstoffe für hydrophobe Gele sind üblicherweise flüssiges Parafin mit Zusatz von Polyethylen oder fetten Ölen, die durch Zusatz von kolloidalem Siliciumdioxid oder Aluminium- oder Zinkseifen geliert werden.

Trägerstoffe für hydrophile Gele sind z.B. Wasser, Glycerin oder Propylenglykol, die mit geeigneten Quellstoffen, wie z.B. Polyacrylsäure, Cellulosederivaten, Stärke, Traganth geliert werden.

Als Emulgatoren für die Herstellung von Emulsionen, Salben und Cremes kommen z.B. in Betracht: Sorbitanester, Wollwachsalkohole, Fettalkohole, Monoglyzeride, Natrium-oder Triethanolaminseifen, Polysorbate oder sulfatierte Fettalkohole.

Die pharmazeutischen Zubereitungen können neben den Wirk-und Trägersubstanzen und den gegebenenfalls vorhandenen Emulgatoren noch andere pharmazeutisch unbedenkliche und mit den Wirkstoffen verträgliche Hilfs-und/oder Zusatzstoffe, wie z.B. Füll-, Streck-, Binde-, Netz-, Stabilisierungs-, Färbe-, Puffer- und Riechstoffe enthalten. Weiterhin können mikrobiologisch aktive chemische Verbindungen, wie

z.B. Konservierungsmittel oder Antiseptica, zur Verbesserung der mikrobiellen Stabilität in den pharmazeutisch üblichen Konzentrationen in den Zubereitungen enthalten sein.

Die erfindungsgemäßen pharmazeutischen Zubereitungen enthalten die erfindungsgemäße Wirkstoffkombination aus Vitamin-E und/oder einem oder mehreren Vitamin-E-Derivaten und Ibuprofen und/oder einem oder mehreren seiner Salze sowie gegebenenfalls einem Zusatz an Salicylsäure und/oder einem oder mehreren Salicylsäurederivaten in einer therapeutisch wirksamen Konzentration. Üblicherweise enthalten die erfindungsgemäßen pharmazeutischen Zubereitungen 1 bis 40 Gew.%, vorzugsweise 5 bis 20 Gew.% wirksame Bestandteile der Wirkstoffkombination. Das gewünschte Verhältnis der wirksamen Bestandteile (Vitamin E und/oder ein oder mehrere Vitamin-E-Derivate) zu (Ibuprofen und/oder einem oder mehreren seiner Salze, gegebenenfalls mit Zusatz von Salicylsäure und/oder einem oder mehrere Salicylsäurederivaten) beträgt dabei, wie bereits erwähnt, normalerweise 1 : (0,025 bis 40), vorzugsweise 1 : (0,25 bis 4), ganz besonders bevorzugt 1 : (0,5 bis 2). Die angegebenen Prozente und Verhältnisse gelten dabei auch, wenn aus den Gruppen Vitamin E/Vitamin E-Derivat, Salicylsäure/Salicylsäurederivat und Ibuprofen/Ibuprofen-Salz jeweils zwei oder mehr Vertreter eingesetzt werden.

Die Herstellung der erfindungsgemäßen pharmazeutischen Zubereitungen erfolgt in an sich bekannter Weise, z.B. durch Mischen der wirksamen Bestandteile mit einem oder mehreren, gegebenenfalls lösenden oder emulgierenden Trägerstoffen und gegebenenfalls einem oder mehreren Zusatz- und/oder Hilfsstoffen. Insbesondere bei der Herstellung von Lösungen oder Emulsionen ist die Zuhilfenahme eines schnellaufenden Rührwerks oder Homogenisators zweckmäßig. Bei der Herstellung der Zubereitungen können auch noch eine oder mehrere andere pharmakologisch wirksame Substanzen zugesetzt werden.

Bei der Verwendung der erfindungsgemäßen Wirkstoffkombination, insbesondere in Form von pharmazeutischen Zubereitungen, zur topischen Behandlung akut-entzündlicher und chronisch-entzündlicher rheumatischer Erkrankungen in Gelenken und Weichteilen sind dabei, sofern keine Salicylsäure-Allergie vorliegt, aufgrund der bisherigen Studien keine gravierenden Nebenwirkungen zu erwarten. Durch topische Applikation direkt über dem Entzündungsherd werden Verdauungstrakt und Pfortaderkreislauf umgangen und somit Verdünnung und vorzeitiger Abbau der wirksamen Substanzen vermieden. Mit Hilfe einer geeigneten, gut penetrierenden Grundlage durchdringen die lipophilen Wirkstoffe schnell und vollständig die Haut und die tieferliegenden Gewebe, so daß ein konzentriertes Anfluten am Ort der rheumatischen Entzündung, z.B. im Bereich der Gelenke, ermöglicht wird.

Die folgenden Ausführungsbeispiele veranschaulichen die Herstellung flüssiger und halbfester erfindungsgemäßer Zubereitungen. Prozentangaben, soweit sie in den folgenden Beispielen vorkommen, beziehen sich immer auf Massenprozente, falls nicht ausdrücklich andere Angaben gemacht werden.

## Beispiel 1

Zur Herstellung von 100 g einer klaren öligen Lösung werden 5 g Ibuprofen und 10 g α-Tocopherolacetat in 85 g Neutralöl (z.B. mittelkettige Triglyceride DAB 9) eingetragen und eventuell unter sehr leichtem Erwärmen bis ca. 30°C gelöst und homogen gerührt. Man erhält ein klares, farbloses Öl, welches in braune Medizinflaschen abgefüllt wird. Anwendung: 3- bis 5mal täglich einreiben.

## Beispiel 2

Zur Herstellung von 100 g einer klaren öligen Lösung werden 2,5 g Ibuprofen und 2,5 g Ethylenglycolsalicylat sowie 12 g α-Tocopherolacetat innig miteinander vermengt und unter Rühren 83 g Neutralöl in kleinen Anteilen hinzugefügt. Das Ibuprofen löst sich dabei langsam, bei Anwendung von gelinder Wärme schnell und vollständig auf. Man erhält ein klares, farbloses Öl, welches in braune Medizingläser abgefüllt wird. Anwendung: 3- bis 5mal täglich an den erkrankten Stellen auftragen und gut einmassieren.

## Beispiel 3

Zur Herstellung einer klaren öligen Zubereitung mit erhöhtem Wirkstoffgehalt werden 5 g Ibuprofen in 15 g 2-Propanol vorgelöst. Dieser Lösung werden unter Rühren nacheinander 5 g Ethylenglycolsalicylat, 12 g α-Tocopherolacetat und 63 g Neutralöl zugesetzt und die Mischung homogen gerührt. Man erhält 100 g einer klaren öligen Lösung. Anwendung: 3mal täglich, in schweren Fällen auch öfter, an den erkrankten Stellen auftragen und einmassieren.

Beispiel 4

Zur Herstellung einer gut haftenden Salbe werden in einer Salben-Reibschale 7 g Ibuprofen in mikronisierter Pulverform mit 14 g α-Tocopherolacetat fein angerieben und in 79 g weißes Vaselin eingearbeitet. Nach dem Homogenisieren, z.B. auf einem Salben-Walzenstuhl, erhält man 100 g einer weißen, konsistenten, geruchlosen Salbe, die in Salbentöpfchen oder Tuben abgefüllt werden kann. Anwendung: 3- bis 4mal täglich, dabei gut einmassieren.

Beispiel 5

Zur Herstellung von 100 g einer cremigen Salbe mit erhöhtem Wirkstoffgehalt werden 10 g mikronisiertes Ibuprofen mit 10 g Neutralöl angerieben, die Anreibung mit 17 g α-Tocopherolacetat vermischt und diese Mischung in 63 g Wollwachsalkoholsalbe (DAB 9) eingearbeitet. Nach dem Homogenisieren erhält man eine weiche, weiße, geruchlose Salbe, die in Kruken, Dosen oder Tuben abgefüllt wird. Anwendung: 3- bis 5mal täglich einreiben und nachmassieren.

Beispiel 6

Zur Herstellung von 100 g einer cremigen Salbe mit variiertem Wirkstoffanteil werden 5 g mikronisiertes Ibuprofen mit 5 g Neutralöl (mittelkettige Triglyceride) fein angerieben und nacheinander mit 5 g Ethylenglycolsalicylat und 10 g α-Tocopherolacetat vermischt. Die Vormischun wird in 75 g Wollwachsalkoholsalbe eingebracht und mit dem Walzenstuhl homogenisiert. Man erhält eine weiche, weiße und geruchlose Salbe, die in Kruken, Dosen oder Tuben abgefüllt werden kann. Anwendung: 3- bis 5mal täglich auf die erkrankten Stellen bringen und ausdauernd einmassieren.

Beispiel 7

Zur Herstellung einer dünnflüssigen Emulsion auf Zellulosebasis werden 2,25 g ®Tylose MH 300 (handelsüblicher wasserlöslicher Zelluloseether der Hoechst AG) unter Rühren in 71,75 g gereinigtes, konserviertes Wasser gegeben und die Mischung 12 h bedeckt zum Quellen stehengelassen. Geeignete Konservierungsstoffe für das Wasser sind z.B. Parahydroxybenzoesäure-ethylester-Natriumsalz oder Kaliumsorbat in Konzentrationen von 0,5 bis 1 Gew.%.

Das Quellgemisch wird mit 1 g Macrogol-1500-glyceroltriricinoleat DAC (als Emulgator) vermischt und danach mit einer geeigneten schnellaufenden Rührmaschine in eine vorgefertige Suspension aus 5 g mikronisiertem Ibuprofen und 15 g Neutralöl eingearbeitet, danach in derselben Weise 5 g α-Tocopherolacetat. Man erhält eine dünnflüssige Emulsion, die in braune Medizinflaschen abgefüllt wird. Anwendung: 4- bis 6mal täglich gründlich einreiben bis das Präparat in die Haut eingezogen ist. Vor Gebrauch schütteln.

Beispiel 8

Zur Herstellung von 100 g eines Emulsionsgels auf Polyacrylatbasis werden 0,625 g Polyacrylsäure DAB 9 in eine Mischung aus 30 g gereinigtem Wasser und 5 g Propylenglycol DAB 9 gebracht und unter Verschluß 3 Tage quellen gelassen. Danach werden unter Rühren 0,07 g Ammoniaklösung 10%ig, 11,305 g gereinigtes Wasser, 5 g Glycerol und 1 g Macrogol-1500-glyceroltriricinoleat zum Ansatz gegeben. Zu dem so erhaltenen Basisgel mischt man eine Lösungvon 5 g Ibuprofen in 32 g 2-Propanol sowie 10 g α-Tocopherolacetat und erzeugt mit Hilfe einer geeig neten Maschine eine feine Emulsion. Man erhält ein weißliches flüssiges Gel, das in Medizinflaschen abgefüllt wird. Anwendung: 4- bis 6mal täglich einmassieren bis das Präparat in die Haut eingezogen ist.

Beispiel 9

Zur Herstellung eines Emulsionsgels mit verstärkter Wirkung werden in 53 g Basisgel nach Beispiel 8 eine Lösung von 5 g Ibuprofen und 5 g Ethylenglycolsalicylat in 22 g 2-Propanol und danach 15 g α-Tocopherolacetat eingearbeitet und anschließend in geeigneter Weise fein emulgiert. Man erhält ein weißes, flüssiges Emulsionsgel, welches in Medizinflaschen aus Glas abgefüllt wird. Anwendung: 4- bis 6mal täglich gründlich einmassieren bis das Präparat in die Haut eingezogen ist.

## Ansprüche

1. Verwendung einer Wirkstoffkombination aus Vitamin E und/oder einem oder mehreren Vitamin E-Derivaten und Ibuprofen und/oder einem oder mehreren seiner Salze, gegebenenfalls mit Zusatz von Salicylsäure und/oder einem oder mehreren Salicylsäurederivaten zur äußerlichen Behandlung von rheumatischen Erkrankungen.

2. Verwendung einer Wirkstoffkombination nach Anspruch 1, dadurch gekennzeichnet, daß die Wirkstoffkombination in einer pharmazeutischen Zubereitung vorliegt und/oder daß in der Wirkstoffkombination das Gewichtsverhältnis von (Vitamin E und/oder einem oder mehreren Vitamin E-Derivaten) zu (Ibuprofen und/oder einem oder mehreren seiner Salze, gegebenenfalls mit einem Zusatz an Salicylsäure und/oder einem oder mehreren Salicylsäurederivaten) = 1 : (0,025 bis 40), vorzugsweise 1 : (0,25 bis 4) und ganz besonders bevorzugt 1 : (0,5 bis 2) beträgt.

3. Verwendung einer Wirkstoffkombination nach den Ansprüchen 1 und/oder 2, dadurch gekennzeichnet, daß in der Wirkstoffkombination das Gewichtsverhältnis (Ibuprofen und/oder einem oder mehreren seiner Salze) : (Salicylsäure und/oder einem oder mehrerer Salicylsäurederivaten) = 1 : (0 bis 200), vorzugsweise 1 : (0 bis 10) und ganz besonders bevorzugt 1 : (0 bis 5) beträgt.

4. Verwendung einer Wirkstoffkombination nach einem oder mehreren der Ansprüche 1 bis 3, dadurch gekennzeichnet, daß die Wirkstoffkombination α-Tocopherol und/oder ein α-Tocopherol-Derivat, Ibuprofen und/oder eines oder mehrerer seiner Salze und gegebenenfalls Ethylenglycolsalicylat (= 2-Hydroxyethylsalicylat) enthält.

5. Wirkstoffkombination zur äußerlichen Behandlung von rheumatischen Erkrankungen, bestehend aus (Vitamin E und/oder einem oder mehreren Vitamin E-Derivaten) und (Ibuprofen und/oder einem oder mehreren seiner Salze, gegebenenfalls mit Zusatz von Salicylsäure und/oder einem oder mehreren Salicylsäurederivaten) neben einem oder mehreren pharmakologisch annehmbaren Trägerstoffen und gegebenenfalls einem oder mehreren Hilfs- und/oder Zusatzstoffen und gegebenenfalls einer oder mehreren zusätzlichen pharmakologisch wirksamen Substanzen.

6. Pharmazeutische Zubereitung zur äußerlichen Behandlung rheumatischer Erkrankungen, dadurch gekennzeichnet, daß sie eine pharmakologisch wirksame Menge einer Wirkstoffkombination aus (Vitamin E und/oder einem oder mehreren Vitamin E-Derivaten) und (Ibuprofen und/oder einem oder mehreren seiner Salze, gegebenenfalls mit Zusatz von Salicylsäure und/oder einem oder mehreren Salicylsäurederivaten) neben einem oder mehreren pharmakologisch annehmbaren Trägerstoffen und gegebenenfalls einem oder mehreren Hilfs- und/oder Zusatzstoffen und gegebenenfalls einer oder mehreren zusätzlichen pharmakologisch wirksamen Substanzen enthält.

7. Pharmazeutische Zubereitung nach Anspruch 6, dadurch gekennzeichnet, daß sie 1 bis 40 Gew.%, vorzugsweise 5 bis 20 Gew.% wirksame Bestandteile der Wirkstoffkombination enthält.

8. Pharmazeutische Zubereitung nach Anspruch 6 und/oder 7, dadurch gekennzeichnet, daß das Gewichtsverhältnis von (Vitamin E und/oder einem oder mehreren Vitamin E-Derivaten) zu (Ibuprofen und/oder einem oder mehreren seiner Salze, gegebenenfalls mit Zusatz von Salicylsäure und/oder einem oder mehreren Salicylsäurederivaten) = 1 : (0,025 bis 40), vorzugsweise 1 : (0,25 bis 4) und ganz besonders bevorzugt 1 : (0,5 bis 2) beträgt.

9. Pharmazeutische Zubereitung nach einem oder mehreren der Ansprüche 6 bis 8, dadurch gekennzeichnet, daß sie α-Tocopherol und/oder ein α-Tocopherol-Derivat und Ibuprofen und/oder ein oder mehrere Salze des Ibuprofens und gegebenenfalls Ethylenglycolsalicylat, enthält.

10. Pharmazeutische Zubereitung nach einem oder mehreren der Ansprüche 6 bis 9, dadurch gekennzeichnet, daß in der Wirkstoffkombination das Gewichtsverhältnis (Ibuprofen und/oder ein oder mehrere Salze des Ibuprofens) : (Salicylsäure und/oder einem oder mehreren Salicylsäurederivaten) = 1 : (0 bis 200), vorzugsweise 1 : (0 bis 10) und ganz besonders bevorzugt 1 : (0 bis 5) beträgt.

11. Verfahren zur Herstellung der in einem oder mehreren Ansprüchen 6 bis 10 angegebenen pharmazeutischen Zubereitungen, dadurch gekennzeichnet, daß Vitamin E und/oder ein oder mehrere Vitamin E-Derivate und Ibuprofen und/oder ein oder mehrere Salze des Ibuprofens gegebenenfalls mit Zusatz von Salicylsäure und/oder einem oder mehreren Salicylsäurederivaten in an sich bekannter Weise mit einem oder mehreren Trägerstoffen und gegebenenfalls mit einem oder mehreren Zusatz- und/oder Hilfsstoffen und gegebenenfalls mit einer oder mehreren anderen pharmakologisch wirksamen Substanzen

gemischt wird.

Europäisches Patentamt

# EUROPÄISCHER RECHERCHENBERICHT

Nummer der Anmeldung

EP 90 10 2342

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | EP-A-0 279 867 (EFEKA FRIEDRICH UND KAUFMANN GmbH)<br>* Seite 11, Zeilen 20-25; Ansprüche 3-5 *<br>--- | 1-11 | A 61 K 31/355<br>A 61 K 31/60 //<br>(A 61 K 31/355<br>A 61 K 31:19 )<br>(A 61 K 31/60<br>A 61 K 31:355<br>A 61 K 31:19 ) |
| A | EP-A-0 152 106 (Dr. ROSHDY ISMAIL)<br>* Seite 5, Zeilen 3-4,6 *<br>--- | 1-11 | |
| A,P | DE-A-3 800 101 (CASSELLA AG)<br>----- | 1-11 | |
| | | | RECHERCHIERTE SACHGEBIETE (Int. Cl.5) |
| | | | A 61 K |

Der vorliegende Recherchenbericht wurde für alle Patentansprüche erstellt

| Recherchenort | Abschlußdatum der Recherche | Prüfer |
|---|---|---|
| DEN HAAG | 09-04-1990 | BRINKMANN C. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)